# EUROPEAN PATENT APPLICATION

(11) **EP 1 723 954 A1**
(43) Date of publication of application: **22.11.2006**
(21) Application number: 06113936.6
(22) Date of filing: 15.05.2006
(51) Int. Cl.: A61K 31/416, A61K 31/4162, A61P 35/00

(54) **Indazole derivatives for the treatment of cancer**

(30) Priority: 18.05.2005 US 132756
(71) Applicant: Carlsbad Technology Inc, Carlsbad, CA 92008 (US)
(72) Inventor: Teng, Che-Ming, 106, Taipei (TW); Kuo, Sheng-Chu, Taichung (TW); Lee, Fang-Yu, Tachia Taichung (TW); Pan, Shiow-Lin, Da-an District Taipei (TW); Guh, Jih-Hwa, 110, Taipei (TW)
(74) Representative: Weihs, Bruno Konrad

(57) **Abstract**

This invention relates to a method for treating cancer including administrating to a subject in need thereof an effective amount of a compound of the formula: 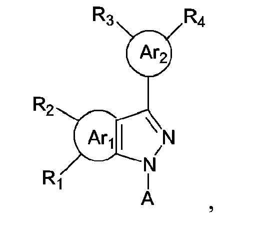 wherein, A is H or 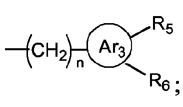 each of Ar₁, Ar₂, and Ar₃, independently, is phenyl, thienyl, furyl, pyrrolyl, pyridinyl, or pyrimidinyl; each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR' , (CH₂)ₘCN, (CH₂)ₘC(O)OR, (CH₂)ₘCHO, (CH₂)ₘCH=NOR, (CH₂)ₘC(O)N(OR)R', N(OR)R', or R₁ and R₂ together, R₃ and R₄ together, or R₅ and R₆ together are O(CH₂)ₘO, in which each of R and R', independently, is H or C₁~C₆ alkyl; and m is 0, 1, 2, 3, 4, 5, or 6, and n is 0, 1, 2, or 3.

## Description

### BACKGROUND

Angiogenesis, formation of new blood vessels, occurs in the healthy body for healing wounds and restoring blood flow to tissues after injury. The angiogenic process is tightly controlled by various positive and negative regulatory factors. In many disease states, the body loses control over angiogenesis.

Excessive blood vessel growth may be triggered by certain pathological conditions such as cancer, age-related macular degeneration, rheumatoid arthritis, and psoriasis. As a result of excessive angiogenesis, new blood vessels feed diseased tissues and destroy normal tissues. In cancer, the new vessels allow tumor cells to escape into the circulation and lodge in other organs.

Angiogenesis occurs via a series of sequential steps, including division and migration of endothelial cells that form the walls of blood vessels. About 15 proteins are known to activate endothelial cell growth and movement. Therefore, angiogenesis can be suppressed by inhibitors of these activating proteins, e.g., angiogenin, epidermal growth factor, estrogen, fibroblast growth factor, interleukin 8, prostaglandins E1 and E2, tumor necrosis factor, vascular endothelial growth factor, or granulocyte colony-stimulating factor.

Excessive angiogenesis-related disorders include cancer (both solid and hematologic tumors), cardiovascular diseases (e.g., atherosclerosis), chronic inflammation (e.g., rheutatoid arthritis or Crohn's disease), diabetes (e.g., diabetic retinopathy), psoriasis, endometriosis, and adiposity. See, e.g., Pharmacological Reviews 52: 237-268, 2001. Compounds that effectively inhibit angiogenesis are drug candidates for treating or preventing these disorders.

### SUMMARY

This invention is based on a surprising discovery that a number of fused pyrazolyl compounds possess anti-angiogenic effect and inhibit the growth of certain cancer cell lines.

Thus, this invention features a method for treating cancer including administrating to a subject in need thereof an effective amount of a compound of the formula: 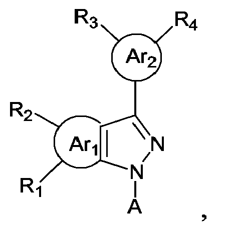 In which A is H or 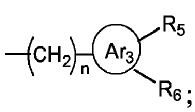 each of Ar₁, Ar₂, and Ar₃, independently, is phenyl, thienyl, furyl, pyrrolyl, pyridinyl, or pyrimidinyl; each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, (CH₂)ₘC(O)N(OR)R', N(OR)R', or R₁ and R₂ together, R₃ and R₄ together, or R₅ and R₆ together are O(CH₂)ₘO, in which each of R and R', independently, is H or C₁~C₆ alkyl, and m is 0, 1, 2, 3, 4, 5, or 6; and n is 0, 1, 2, or 3. (CH₂)ₘ can be branched or linear. Note that the left atom shown in any substituted group described above is closest to the fused pyrazolyl ring. Also note that when there are one or more R or (CH₂)ₘ moieties in a fused pyrazolyl compound, the R or the (CH₂)ₘ moieties can be the same or different.

A subset of the above-described compounds are those in which A is 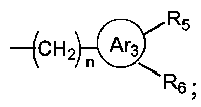 each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO. In some embodiments, each of Ar₁, Ar₂, and Ar₃ is phenyl or furyl and each of R₁, R₂, R₅, and R₆ is H , halo, or C₁~C₆ alkyl, and n is 1.

Another subset of the above-described compounds are those in which A is 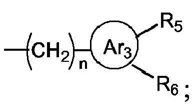 each of R₁, R₂, R₃, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (O)OR, (CH₂)ₘCHO, (CH₂)ₘCH=NOR, or R₁ and R₂ together, or R₅ and R₆ together are O(CH₂)ₘO; and R₄ is (CH₂)ₘC(O)N(OR)R' or N(OR)R'. In some embodiments, each of Ar₁, Ar₂, and Ar₃ is phenyl or furyl; and each of R₁, R₂, R₅, and R₆ is H, halo, or C₁~C₆ alkyl; and n is 1.

Still another subset of the above-described compounds are those in which A is H. In some embodiments, each of Ar₁ and Ar₂ is phenyl or furyl; and each of R₁, R₂, and R₃ is H, and R₄ is CH₂(OH) or CO₂CH₃.

Examples of cancer that can be treated by the method described above include leukemia, colorectal cancer, prostate cancer, lung cancer, breast cancer, and renal cancer.

Shown below are exemplary compounds used in this invention:

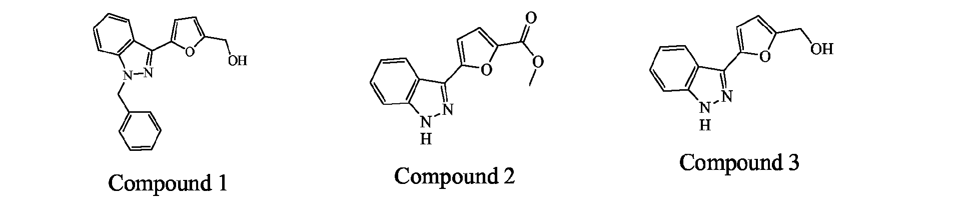

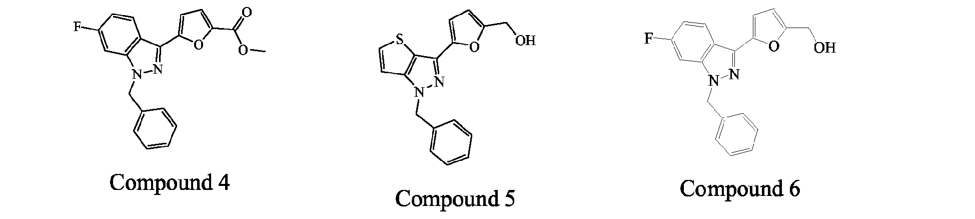

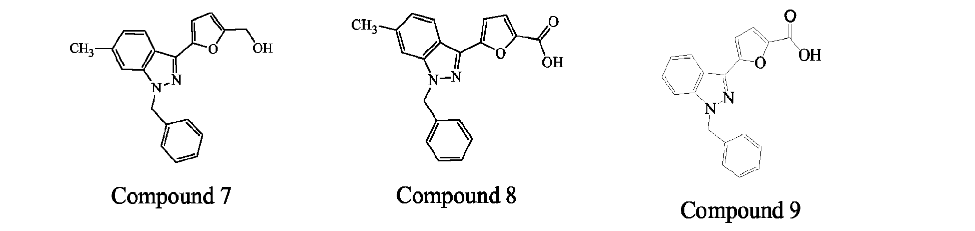

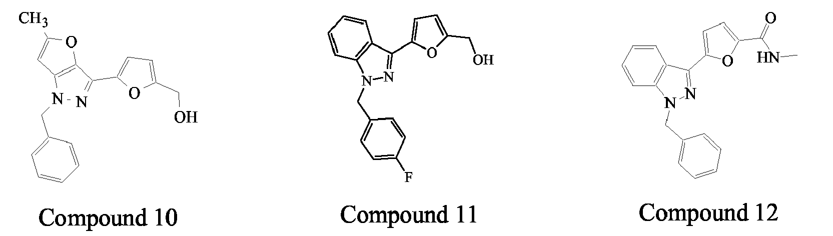

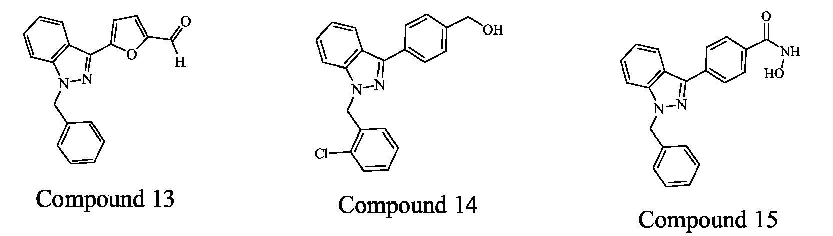

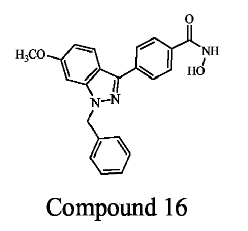

The term "Ar," as used herein, refers to both aryl and heteroaryl groups. Aryl, e.g., phenyl, is a hydrocarbon ring system having at least one aromatic ring. Heteroaryl is a hydrocarbon ring system having at least one aromatic ring which contains at least one heteroatom such as O, N, or S. Examples of heteroaryl include, but are not limited to, thienyl, furyl, pyrrolyl, pyridinyl, and pyrimidinyl. An "Ar" may contain one, two, three, or more substituents on its ring. In addition to those assigned to R₁, R₂, R₃, R₄, R₅, and R₆ (see above), the substituents can also be nitro, C₂-C₆ alkenyl, C₂~C₆ alkynyl, aryl, heteroaryl, cyclyl, or heterocyclyl. Alkyl, alkenyl, alkynyl, alkoxy, aryl, heteroaryl, cyclyl, and heterocyclyl, as used herein, are optionally substituted with C₁~C₆ alkyl, halogen, amino, hydroxyl, mercapto, cyano, or nitro. Note that the term "alkyl" refers to both linear alkyl and branched alkyl.

The fused pyrazolyl compounds described above include the compounds themselves, as well as their salts and their prodrugs, if applicable. Such salts, for example, can be formed by interaction between a negatively charged substituent (e.g., carboxylate) on a fused pyrazolyl compound and a cation. Suitable cations include, but are not limited to, sodium ion, potassium ion, magnesium ion, calcium ion, and an ammonium cation such as teteramethylammonium ion. Likewise, a positively charged substituent (e.g., amino) can form a salt with a negatively charged counterion. Suitable counterions include, but are not limited to, chloride, bromide, iodide, sulfate, nitrate, phosphate, or acetate. Examples of prodrugs include esters and other pharmaceutically acceptable derivatives, which, upon administration to a subject, are capable of providing the fused pyrazolyl compounds described above.

Also within the scope of this invention are a composition containing one or more of the fused pyrazolyl compounds described above for use in treating cancer, and the use of such a composition for the manufacture of a medicament for cancer.

Other features, objects, and advantages of the invention will be apparent from the description and from the claims.

### DETAILED DESCRIPTION

A fused pyrazolyl compound used to practice the method of this invention can be prepared by procedures well known to a skilled person in the art (see, e.g., U.S. Patent No. 5,574,168). They include the following synthetic route: An aryl aryl ketone is first prepared by coupling an arylcarbonyl chloride with another aryl compound. Either aryl compound is optionally mono- or multi-substituted. The ketone then reacts with an arylalkylhydrazine, the aryl group of which is also optionally mono- or multi-substituted, to form a hydrazone containing three aryl groups. The hydrazone group is transformed into a fused pyrazolyl core via an alkylene linker, another aryl group is fused at 4-C and 5-C of the pyrazolyl core, and the third aryl group is directly connected to 3-C of the pyrazolyl core. Derivatives of the fused pyrazolyl compound may be obtained by modifying the substituents on the aryl groups via known transformations. For example, a methoxylcarbonyl group (-CO₂Me) is transformed into a hydroxymethyl group (-CH₂OH) by a suitable reducing agent. As another example, a methoxycarbonyl group is hydrolyzed to a carboxylic acid (-COOH), which is subsequently converted to a more reactive group, acyl halide (-C(O)X). The acyl halide can be reacted with ammonium or hydroxylamine to form an amide group (-C(O)NH₂) or a hydroxamic acid group (-C(O)NHOH).

The chemicals used in the above-described synthetic route may include, for example, solvents, reagents, catalysts, protecting group and deprotecting group reagents. The methods described above may also additionally include steps, either before or after the steps described specifically herein, to add or remove suitable protecting groups in order to ultimately allow synthesis of the fused pyrazolyl compound. In addition, various synthetic steps may be performed in an alternate sequence or order to give the desired compounds. Synthetic chemistry transformations and protecting group methodologies (protection and deprotection) useful in synthesizing applicable fused pyrazolyl compounds are known in the art and include, for example, those described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

A fused pyrazolyl compound thus synthesized can be further purified by a method such as column chromatography, high pressure liquid chromatography, or recrystallization.

This invention features a method for treating cancer including administering to a subject in need thereof an effective amount of one or more fused pyrazolyl compounds and a pharmaceutically acceptable carrier. The term "treating" is referred to as application or administration of a composition including one or more fused pyrazolyl compounds to a subject, who has cancer, a symptom of cancer, or a predisposition toward cancer, with the purpose to cure, heal, alleviate, relieve, alter, remedy, ameliorate, improve, or affect the cancer, the symptoms of the cancer, or the predisposition toward the cancer. "An effective amount" is referred to as the amount of a fused pyrazolyl compound which, upon administration to a subject in need thereof, is required to confer therapeutic effect on the subject. Effective amounts may vary, as recognized by those skilled in the art, depending on route of administration, excipient usage, and the possibility of co-usage with other agents..

As used herein, "cancer" is referred to cellular tumor. Cancer cells having the capacity for autonomous growth, i.e., an abnormal state or condition characterized by rapidly proliferating cell growth. The term is meant to include all types of cancerous growths or oncogenic processes, metastatic tissues or malignantly transformed cells, tissues, or organs, irrespective of histopathologic type, or stage of invasiveness. Examples of cancers include, but are not limited to, carcinoma and sarcoma such as leukemia, sarcomas, osteosarcoma, lymphomas, melanoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, endometrial cancer, nasopharyngeal cancer, cervical or hepatic cancer, or cancer of unknown primary site.

To practice the method of the present invention, a fused pyrazolyl compound can be administered orally, parenterally, by inhalation spray, or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

A composition for oral administration can be any orally acceptable dosage form including, but not limited to, tablets, capsules, emulsions and aqueous suspensions, dispersions and solutions. Commonly used carriers for tablets include lactose and com starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

An inhalation composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A carrier in a pharmaceutical composition must be "acceptable" in the sense of being compatible with the active ingredient of the formulation (and preferably, capable of stabilizing it) and not deleterious to the subject to be treated. For example, solubilizing agents, such as cyclodextrins (which form specific, more soluble complexes with fused pyrazolyl compounds), can be utilized as pharmaceutical excipients for delivery of fused pyrazolyl compounds. Examples of other carriers include colloidal silicon dioxide, magnesium stearate, cellulose, sodium lauryl sulfate, and D&C Yellow # 10.

A suitable *in vitro* assay can be used to preliminarily evaluate the efficacy of a fused pyrazolyl compound in inhibiting the activities of fibroblast growth factor (FGF) or vascular endothelial growth factor (VEGF). *In vivo* assays can also be performed by following procedures well known in the art to screen for efficacious fused pyrazolyl compounds. See the specific examples below.

Similarly, an suitable *in vitro* assay can be used to preliminarily evaluate the efficacy of a fused pyrazolyl compound in inhibiting the growth of cancer cells. The fused pyrazolyl compound can further be examined for its efficacy in treating cancer by *in vivo* assays. For example, the compound can be applied to an animal (e.g., a mouse model) having cancer and its therapeutic effects are then accessed. Based on the results, an appropriate dosage range and administration route of the pyrazolyl compound can also be determined.

Without further elaboration, it is believed that the above description has adequately enabled the present invention. The following specific embodiments are, therefore, to be construed as merely illustrative, and not limitative of the remainder of the disclosure in any way whatsoever. All of the publications, including patents, cited herein are hereby incorporated by reference in their entirety.

### Synthesis of 1-benzyl-3-(5'-hydroxymethyl-2'-furyl)indazole (Compound 1)

Calcium borohydride was first prepared by stirring anhydrous calcium chloride (88.8 mg, 0.8 mmole) with sodium borohydride (60 mg, 1.6 mmole) in anhydrous THF (20 mL) for 4 hrs. Then a 30 mL THF solution containing 88.0 mg 1-benzyl-3-(5'-methoxycarbonyl-2'-furyl)indazole (0.27 mmole) was added dropwise to the calcium borohydride solution at 30±2 °C. The mixture was heated under reflux for 6 hrs, cooled, quenched into crushed ice, placed at a reduced pressure to remove THF, and filtered to obtain a solid product. The solid was extracted with dichloromethane. The extract was concentrated to 50 mL and a solid precipitated after petroleum ether was added. The precipitate was collected and purified by column chromatography (silica gel-benzene) to obtain 70.0 mg 1-benzyl-3-(5'-hydroxymethyl-2'-furyl)indazole at a yield of 87%. mp: 108-109°C.
MS (%), m/z: 304 (M⁺).
IR (KBr) vₘₐₓ: 3350 cm⁻¹ (-OH).
¹H-NMR (DMSO-d₆, 200 MHz) δ: 4.51 (2H, d, J=5.5 Hz, -CH₂O-) 5.31 (1H, t, J=5.5 Hz, -OH), 5.70 (2H, s, =NCH₂-), 6.48 (1H, d, J=3.4 Hz, H-4'), 6.97 (1H, d, J=3.4 Hz, H-3'), 7.21-7.31 (6H, m, H-5, phenyl), 7.45 (1H, t, J=8.2 Hz, H-6), 7.75 (1H, dd, J=8.2, 1.8 Hz, H-7), 8.12 (1H. dd, J=8.2, 1.0 Hz, C4-H).

### Inhibition of DNA synthesis

Human umbilical vein endothelial cells (HUVECs) were incubated in the absence of Compound 1 (basal and control) or presence of Compound 1 (with a concentration of 0.1 TM, 0.03 TM, 0.1 TM, 0.3 TM, or 1 TM). Vascular endothelial growth factor (VEGF) or basic fibroblast growth factor (bFGF) was added (except for basal) to induce DNA synthesis, which was detected based on [³H]thymidine incorporation. The results show that Compound 1 inhibited VEGF- and bFGF-induced cell proliferation of HUVECs in a concentration-dependent manner. Unexpectedly, Compound 1 had IC₅₀ values of 9.0 × 10⁻⁸ M and 1.4 × 10⁻⁷ M, for VEGF and bFGF, respectively.

Additional 23 fused pyrazolyl compounds were also tested. All of them inhibited VEGF-induced cell proliferation of HUVECs, some as potent as Compound 1.

### Inhibition of tube formation

HUVECs were cultured onto chamberslide, which was pre-coated with Matrigel (10 mg/mL). Cells were treated without Compound 1 (control) or with Compound 1 (10 TM). VEGF (10 ng/mL) or bFGF (10 ng/mL) was added to induce tube formation. All photos were taken at 100X magnification. The results show that Compound 1 inhibited VEGF- and bFGF-induced formation of networks of elongated endothelial cells.

### Inhibition of angiogenic effect

Nude mice were subcutaneously injected with a Matrigel plug containing 150 ng/mL VEGF or bFGF. Vehicle or Compound 1 was administrated to the mice orally (1 mg/kg/day, 3 mg/kg/day, 10 mg/kg/day, 30 mg/kg/day, or 100 mg/kg/day) for seven days. The angiogenic response was monitored visually through the transparent skin. Matrigel itself did not elicit an angiogenic response. After seven days the mice were sacrificed and the Matrigel plugs were observed in situ to quantify the ingrowth of blood vessels. The plugs were removed, fixed in 4% formaldehyde, embedded in paraffin, sectioned at 5-Tm thick for histological analysis, and blood vessel growth quantitated by hematoxylin-eosin staining. All photos were taken at 40X magnification. The results show that oral administration of Compound 1 for seven days effectively inhibited VEGF or bFGF-induced angiogenic effect in a dose-dependent manner.

In a quantitative analysis of angiogenic effect, nude mice were treated as described above, and the plugs were removed and dissolved. Hemoglobin concentrations were measured using a hemoglobin detection kit (Sigma Chem. Co.) as indices of angiogenesis. The results illustrates that Compound 1 effectively inhibited VEGF or bFGF-induced angiogenic effect.

### Inhibitory effect on cancer growth

BALB/c-nu mice were subcutaneously injected with A549 lung tumor cells (10⁷ cell/mouse). After inoculation for 29 days, each tumor was grown to a size of 30 to 50 mm³. The mice were divided into two groups. A number ofpyrrazolyl compounds were each dissolved in 0.5% carboxymethyl cellulose to provide sample solutions (1 mg/ml). The solutions were each orally administered to the mice every day (for pyrrazolyl compounds 10 mg/kg/day) from the 29^{th} day, and the tumor sizes were measured every 3 to 4 days. The mice were euthanatized with intraperitoneal administration of pentobarbital at the 53^{rd} day. The tumors were removed and weighed.

The results show that the mice treated with Compounds 1, 12, and 14 had unexpectedly smaller tumors than that treated with the vehicle.

A number of pyrrazolyl compounds were also tested, following procedures similar to that described above, for their inhibitory effect on the growth of other cancer cells, i.e., NCI-H226 (lung cancer cells), A 498 (renal cancer cells), ACHN (renal cancer cells), UO-31 (renal cancer cells), HA22T (hepatoma cells), HT-29 (colorectal cancer cells), HCT-116 (colorectal cancer cells), PC-3 (prostate cancer cells), MDA-MB-468 (breast cancer cells), and CCRF-CEM (leukemia cells).

The results show that Compounds 1, 10, 11, 15, and 16 effectively inhibited the growth of NCI-H226 cells, Compounds 1, 3, 5-7, 11, and 13-16 effectively inhibited the growth of A 498 cells, Compound 2 effectively inhibited the growth of ACHN cells, Compounds 1, 2, 4, and 6 effectively inhibited the growth of UO-31 cells, Compound 7 effectively inhibited the growth of HT-29 cells, Compounds 1, 2, and 5 effectively inhibited the growth of HCT-116 cells, and Compounds 1, 4, and 6-8 effectively inhibited the growth of PC-3 cells, Compound 1 effectively inhibited the growth of MDA-MB-468 cells, and Compounds 1, 3, 4, 6, 8, and 9 effectively inhibited the growth of CCRF-CEM cells. Unexpectedly, Compound 1 inhibited growth of A498 cells, NCI-H226 cells, and MCF-7 cells more effectively than HA22T cells.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. For example, a compound structurally analogous to a fused pyrazolyl compound can also be used to practice the present invention. Thus, other embodiments are also within the claims.

## Claims

1. A method for treating cancer, comprising administrating to a subject in need thereof an effective amount of a compound of the formula: 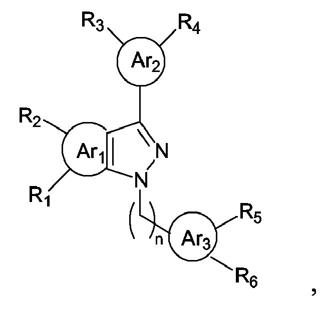 wherein
each of Ar₁, Ar₂, and Ar₃, independently, is phenyl, thienyl, furyl, pyrrolyl, pyridinyl, or pyrimidinyl;
each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO, in which each of R and R', independently, is H or C₁~C₆ alkyl, and m is 0, 1, 2, 3, 4, 5, or 6; and
n is 0, 1, 2, or 3;
the cancer being leukemia, colorectal cancer, prostate cancer, lung cancer, breast cancer, or renal cancer.

2. The method of claim 1, wherein Ar₁ is phenyl.

3. The method of claim 2, wherein Ar₂ is 5'-furyl.

4. The method of claim 3, wherein Ar₃ is phenyl.

5. The method of claim 4, wherein each of R₁, R₂, R₅, and R₆ is H.

6. The method of claim 5, wherein n is 1.

7. The method of claim 6, wherein one of R₃ and R₄ is substituted at position 2 of furyl.

8. The method of claim 7, wherein one of R₃ and R₄ is H, and the other is CH₂OH.

9. The method of claim 1, wherein Ar₂ is 5'-furyl.

10. The method of claim 9, wherein one of R₃ and R₄ is substituted at position 2 of furyl.

11. The method of claim 10, wherein Ar₃ is phenyl.

12. The method of claim 1, wherein the cancer is leukemia.

13. The method of claim 1, wherein the cancer is colorectal cancer.

14. The method of claim 1, wherein the cancer is prostate cancer.

15. The method of claim 1, wherein the cancer is lung cancer.

16. The method of claim 1, wherein the cancer is breast cancer.

17. The method of claim 1, wherein the cancer is renal cancer.

18. The method of claim 8, wherein the cancer is renal cancer.

19. The method of claim 8, wherein the cancer is lung cancer.

20. The method of claim 8, wherein the cancer is lung cancer.

21. A method for treating cancer, comprising administrating to a subject in need thereof an effective amount of a compound of the formula: 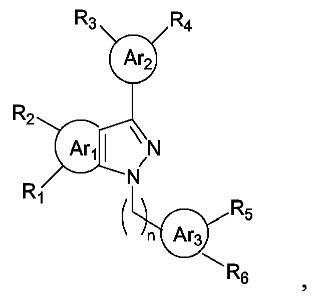 wherein
each of Ar₁, Ar₂, and Ar₃, independently, is phenyl, thienyl, furyl, pyrrolyl, pyridinyl, or pyrimidinyl;
each of R₁, R₂, R₃, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (O)OR, (CH₂)ₘCHO, (CH₂)ₘCH=NOR, or R₁ and R₂ together, or R₅ and R₆ together are O(CH₂)ₘO; and R₄ is (CH₂)ₘC(O)N(OR)R' or N(OR)R'; in which each ofR and R', independently, is H or C₁~C₆ alkyl, and m is 0, 1, 2, 3, 4, 5, or 6; and
n is 0, 1, 2, or 3.

22. The method of claim 21, wherein the cancer being leukemia, colorectal cancer, prostate cancer, lung cancer, breast cancer, or renal cancer.

23. The method of claim 22, wherein Ar₁ is phenyl.

24. The method of claim 23, wherein n is 1 and Ar₃ is phenyl.

25. The method of claim 24, wherein Ar₂ is phenyl.

26. The method of claim 25, wherein R₄ is C(O)N(OR)R'.

27. The method of claim 21, wherein the cancer is lung cancer or renal cancer.

28. A method for treating cancer, comprising administrating to a subject in need thereof an effective amount of a compound of the formula: 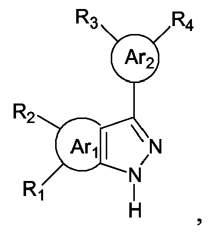 wherein
each of Ar₁ and Ar₂, independently, is phenyl, thienyl, furyl, pyrrolyl, pyridinyl, or pyrimidinyl;
each of R₁, R₂, R₃, and R₄, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, (CH₂)ₘC(O)N(OR)R', or N(OR)R' or R₁ and R₂ together or R₃ and R₄ together are O(CH₂)ₘO, in which each of R and R', independently, is H or C₁~C₆ alkyl, and m is 0, 1, 2, 3, 4, 5, or 6; and
n is 0, 1, 2, or 3.

29. The method of claim 28, wherein the cancer being leukemia, colorectal cancer, prostate cancer, lung cancer, breast cancer, or renal cancer.

30. The method of claim 28, wherein Ar₁ is phenyl.

31. The method of claim 30, wherein Ar2 is 5'furyl.

32. The method of claim 31, wherein each of R₁, R₂, and R₄ is H, and R₃ is CH₂(OH) or CO₂CH₃.

33. The method of claim 32, wherein the cancer is leukemia, renal cancer, hepatoma, or colorectal cancer.
